# EUROPEAN PATENT SPECIFICATION

(11) **EP 1 185 268 B1**
(45) Date of publication and mention of the grant of the patent: **17.08.2005**
(21) Application number: 00940504.4
(22) Date of filing: 08.06.2000
(51) Int. Cl.: A61K 31/4458, A61P 25/00

(54) **THE THERAPEUTIC USE OF D-THREO-METHYLPHENIDATE FOR THE TREATMENT OF ATTENTION-DEFICIT HYPERACTIVITY DISORDER**
THERAPEUTISCHE VERWENDUNG VON D-THREO-METHYLPHENIDATE ZUR BEHANDLUNG VON AUFMERKSAMKEITSMANGEL-HYPERAKTIVITÄTSSTÖRUNGEN
UTILISATION THERAPEUTIQUE DE i D-THREO /i -METHYLPHENIDATE POUR LE TRAITEMENT DE TROUBLES D'HYPERACTIVITE DEFICITAIRES DE L'ATTENTION

(30) Priority: 09.06.1999 GB 9913458
(43) Date of publication of application: 13.03.2002
(73) Proprietor: Celltech Pharma Europe Limited, Slough, Berkshire SL1 3WE (GB)
(72) Inventor: Davidson, Elizabeth Janina, Celltech Pharma Ltd, Berks, SL1 3WE (GB)
(74) Representative: Perry, Robert Edward
(86) International application number: PCT/GB2000/002234
(87) International publication number: WO 2000/074680

(56) References cited:
- WO-A-97/03673
- WO-A-97/27176
- WO-A-99/03471
- WO-A-99/16439
- WO-A-99/30694
- US-A- 5 908 850
- US-A- 6 025 502
- SRINIVAS NUGGEHALLY R; HUBBARD JOHN W; QUINN DECLAN; MIDHA KAMAL K: "Enantioselective pharmacokinetics and pharmacodynamics of racemic-threo-methylphenidate in children with attention deficit hyperactivity disorder." CLINICAL PHARMACOLOGY & THERAPEUTICS , vol. 52, no. 5, 1992, pages 561-568, XP000950002 cited in the application
- JONKMAN L M ET AL: "Differences in plasma concentrations of the D- and L-threo methylphenidate enantiomers in responding and non- responding children with attention - deficit-- hyperactivity disorder." PSYCHIATRY RESEARCH, (1998 MAR 20) 78 (1-2) 115-8. , XP000949999 cited in the application

## Description

### Field of the Invention

This invention relates to the therapeutic use of *d*-*threo*-methylphenidate.

### Background of the Invention

To date, the literature has indicated that the pharmacological action of *d,l-threo*-methylphenidate (*d,l*-MPH; available as Ritalin®) in the treatment of attention-deficit hyperactivity disorder (ADHD) is the property of the *d*-enantiomer (*d*-MPH), as no action on the part of the /-enantiomer (/-MPH) has been detected; see Patrick (1987), and Srinivas (1992). It has also been found that, following oral dosing, the *l*-enantiomer is metabolised preferentially such that plasma levels of the *d*-enantiomer are generally found to be higher than those of the /-enantiomer, and very little /-MPH enters the circulation or becomes available to the brain: see Aoyama *et al*, Eur. J. Clin. Pharmacol. 44:79-84 (1993), and Hubbard, J. Pharm. Sci. 78:944-7 (1989).

Intravenous administration of *d*,/-MPH has shown similar plasma levels of the two enantiomers for around 1.5 hours after dosing, after which the levels diverge (Srinivas, 1993). Ding *et al,* Psychopharmacology 131:71-78 (1997), shows that /-MPH is detected in the brain after intravenous dosing.

Ding *et al, supra,* also suggests (as indicated above) that the major pharmacological activity of racemic methylphenidate lies in the *d*-MPH moiety. *l*-MPH appears relatively inactive in behavioural models; see Eckerman *et al,* Pharmacol. Biochem. Behav. 40:875-880 (1991).

Bames *et al*, Eur. J. Pharmacol. 218:15-25 (1992), reports that enantiomers of zacopride interact with PCPA, to modify rodent aversive behaviour and the less active enantiomer has been found to antagonise the activity of the more active.

Approximately one-third of children with ADHD do not respond clinically with *d,l*-MPH. Jonkman *et al,* Psychiatry Research 78:115-8 (1998), proposed that metabolic differences might account for the lack of response, and found that non-responders had higher plasma levels of both enantiomers than did responders.

### Summary of the Invention

The present invention is based on new evidence (presented below) that *d*-MPH may be used to treat non-responders to racemic MPH.

Firstly, it has surprisingly been found that *l*-MPH possesses pharmacological activity broadly similar to that of the *d*-enantiomer and the racemate. The two enantiomers and the racemate induced similar stimulant effects in the Irwin Observation Test (see Irwin, Psychopharm. 131:71-8 (1968)), including mainly excitation with signs of hypersensitivity to external stimulation, stereotypies with fore-paw treading, mydriasis and hyperthermia. The *l*-enantiomer was approximately one-eighth as active as the *d*-enantiomer and one-quarter as active as *d,l*-MPH and is, therefore, a partial agonist.

A second piece of evidence is based on a study, in the mouse, that tested the ability of *l*-MPH to antagonise the effects of *d*-MPH or *d,l*-MPH on locomotor activity. It was found that *l*-MPH dosed subcutaneously at 25 mg/kg followed by *d,l*-MPH at 5 mg/kg resulted in a reduction in locomotor activity, compared with a control group dosed with saline followed by *d,l*-MPH.

Without wishing to be bound by theory, it is suggested that *l*-MPH within the racemate might antagonise the action of *d*-MPH, provided that sufficient *l*-MPH enters the brain. Further, it is deduced that the difference in the metabolism of MPH in non-responders leads to sufficient /-MPH entering the circulation and becoming available to the brain, to block the receptors to the *d*-MPH and to account for the non-response. The consequence is that a formulation consisting essentially only of the single *d*-enantiomer would not encounter any antagonism and could have positive actions in non-responders to *d,l*-MPH; alternatively, if it cannot be shown that the *l*-enantiomer is a direct antagonist *via* the mechanism of receptor-blockade, the *d*-enantiomer might have a more potent pharmacological action in the absence of the /-enantiomer, by removal of the pharmacological effects of the latter and any interference resulting therefrom.

### Description of the Invention

The *d*-MPH that is used in this invention is substantially free of its antipode (*l*-MPH), e.g. in an enantiomeric excess (ee) of at least 70%, preferably at least 90%, and more preferably at least 95%. The *d*-MPH may be substantially enantiopure It may be used in the form of any suitable salt, e.g. the hydrochloride.

The *d*-MPH may be administered by the same means as is known for racemic methylphenidate, in an immediate, modified or sustained-release formulation, e.g. a coated tablet, or as a liquid. It may be administered in any other conventional formulation, via any suitable route of administration. Conventional dosing parameters may be adopted, i.e. those which are known to or adapted to the practice of those skilled in the art. Examples of suitable compositions are disclosed in WO-A-9703673.

Compositions of the invention may be administered for known purposes, e.g. the treatment of attention-deficient hyperactivity disorder (ADHD; this term is used herein to encompass attention-deficit disorder) in pre-pubertal children, adolescents and in adults, as a stimulant in cancer patients treated with narcotic analgesics, and also for the treatment of depression (e.g. in AIDS patients), compulsive shopping disorder, narcolepsy and hypersomnia.

The patient may be any that has been identified as a "non-responder". This represents a class of patients that is already known, or patients that can readily be identified by the skilled person. See Jonkman *et al, supra.*

Typically, the patient will be adolescent or pre-adolescent. The age of the patient may be, for example, from 5 to 15 years. However, adults may also be suitable for treatment according to this invention.

Evidence, on which the invention is based, comprises the following studies.

### Study 1

This study was designed to examine the general clinical effects (Irwin test) and anticonvulsant activity of /-MPH and its effects on barbital-induced sleep, intestinal transit and gastric function in the rat. For the Irwin test, intestinal transit and gastric function, it was compared with the *d*-enantiomer and the racemate.

More specifically, the test and reference compounds were *l*-MPH hydrochloride, saltlbase ratio = 1.163, dissolved in physiological saline; *d*-MPH hydrochloride, salt/base ratio = 1.163, dissolved in physiological saline; *d,l*-MPH hydrochloride, salt/base ratio = 1.163, dissolved in physiological saline; caffeine (barbital test), diazepam (barbital and PTZ tests), RO 15-4513 (PTZ test), morphine (intestinal transit test) and cimetidine (gastric acid secretion test). The control was vehicle (physiological saline).

Male Rj Wistar (Han) rats (5 or 6 per cage), 180-240 g body weight range, were stabilised for at least 5 days after delivery in cages (41 x 25 x 14 cm) on wood shavings. The animals had free access to food and tap water until tested.

The treatment schedules were:
Irwin test: 2, 4, 8, 16, 32, 64, 128 and 256 mg/kg s.c. immediately before the test;
Barbital and pentylenetetrazole tests: 8, 16 and 32 mg/kg s.c. 30 minutes before the test;
Intestinal transit and gastric secretion tests: 16 mg/kg s.c. 30 minutes before the test.

Results showed that the two enantiomers and the racemate induced similar effects in the Irwin observation test, including mainly excitation with signs of hypersensitivity to external stimulation, stereotypies with fore-paw treading, mydriasis and hyperthermia. Clear stimulant effects were observed from 16 mg/kg for the *l*-enantiomer, from 2 mg/kg for the d-enantiomer and from 4 mg/kg for the racemate. *l*-MPH was not lethal up to 256 mg/kg. In contrast, the d-enantiomer and the racemate induced lethality at 64 mg/kg.

*l*-MPH completely antagonised barbital-induced sleep and clearly and dose-dependently antagonised the convulsive effects of pentylenetetrazole. It also decreased intestinal transit, gastric fluid volume and gastric acidity.

Similar effects were observed with the d-enantiomer and the racemate in the intestinal transit and the gastric acid secretion tests. On the one hand, the decrease in intestinal transit tended to be less pronounced than that observed with the *l*-enantiomer (-12% and -15% respectively as compared with -25%). On the other hand, the effects of the racemate on gastric acid secretion and gastric fluid pH appeared more marked than those observed with either enantiomer.

Taken together, these results indicate clear CNS stimulant and anticonvulsant effects for *l*-MPH in the rat in the dose-range 8-32 mg/kg s.c. They also indicate a decrease in intestinal transit and gastric acid secretion with this substance (16 mg/kg). However, the latter effects remained slight as compared with the reference substances morphine and cimetidine.

No clear differences were observed between the enantiomers and the racemate in any of the tests used, apart from the Irvvin test, when the *d*-enantiomer was approximately 8-times more potent than the *l*-enantiomer, intermediate potency being observed for the racemate.

### Study 2 (Conditioned Place Preference)

Adult male BKW (Bradford bred) mice weighing 34-50 g at the start of the study were used. Mice were housed in groups of 10 in a holding room maintained at 21 ± 2°C, under reversed lighting conditions (12:12 h; lights on 19.00-07.00 h). Food and waterwere available *ad libitum* except during testing in the behavioural apparatus. On each day of the study, mice were transported to the experimental room in an enclosed trolley, at least 1 h before testing commenced. The experimental room was maintained in red lighting.

In a preliminary dose ranging study using locomotor activity (LMA) assessment, apparatus consisted of 15 individual clear plexiglass cages (10 x 24 x 14 cm), each fitted with two photocell units located 2.5 cm above the floor of the cage and 3 cm from the long sides. Interruptions of the light beams were recorded automatically and are presented as total counts/time period. A preliminary study was performed to establish the overt effects of *l*-MPH using a cumulative dosing technique as follows. Naive mice (n = 5/group) received saline or *l*-MPH (6.25 mg/kg, s.c.) and were placed immediately into the LMA apparatus. 30 min later a second dose of saline or *l*-MPH (6.25/kg, s.c.) was administered, and the mice returned to the LMA apparatus. Following a further 30 min period, a third dose of saline or /-MPH (12.5 mg/kg, s.c.) was administered and LMA was recorded. Finally, 30 min later, a fourth dose of saline or I-MPH (25 mg/kg, s.c.) was administered and LMA was recorded for 30 min.

A summary of the data obtained is presented in Table1. From these data, it was concluded that the conditions place preference (CPP) experiment would proceed using *l*-MPH at 6.25, 12.5 and 25 mg/kg, s.c. since behavioural effects were minimal (possibly as a result of the combination of the experimental design and low numbers of animals used) and no overt signs of toxicity were observed.

**Table 1**

| Treatment (mg/kg, s.c.) | Locomotor activity (counts/30 min) |
|---|---|
| Saline (First dose, time 0) | 721.6 ± 143.6 |
| *l*-MPH (6.25) | 1025.0 ± 117.0 |
| Saline (Second dose, 30 min.) | 408.4 ± 118.0 |
| *l*-MPH (6.25) | 703.6 ± 114.2 |
| Saline (Third dose, 60 min.) | 240.0 ± 77.6 |
| *l*-MPH (12.5) | 646.2 ± 188.5* |
| Saline (Fourth dose, 90 min.) | 111.8 ± 67.7 |
| /-MPH(25) | 320.6 ± 114.1 |

| | |
|---|---|
| n = 5/treatment group. Data are presented as mean ± sem. *P<0.05 (ANOVA followed by Dunnett's t-test): significant increase from the corresponding saline control. | |

### CPP Study

CPP was assessed in a 3-chambered apparatus (76 x 30 x 30 cm) constructed from Plexiglass. The outer two chambers measured 30 x 30 x 30 cm, one with a striped wood floor/metal walls and the other with a textured glass floor/striped wood walls. This combination of textures and visual clues has been chosen since it ensures that the two chambers are distinct. Mice are allocated to the initially preferred and non-preferred chamber using a counterbalanced design. The smaller central chamber (16 x 30 x 30 cm) consists of a permanently black painted floor with clear walls. All three chambers are connected by guillotine doors, which are staggered to prevent visual communication between the chambers.

The experimental session was divided into three separate phases.

### Pre-conditioning:

Naive mice were placed in the central chamber, with the guillotine doors raised, and allowed free access to all three sections of the apparatus for 15 min on three consecutive days. The position of the mouse in the apparatus was monitored automatically using a system of photocell beams and the time spent (s) in each of the two outer chambers was recorded, from which the pre-conditioning preference was determined (mean ± sem of the 3 days). The time spent in the central chamber reflects the number of transitions between the two outer chambers.

### Conditioning:

This consisted of a period of 8 days in which the guillotine doors were lowered and the mice (groups n = 7-12) were injected with drug or saline and placed immediately into one of the two outer chambers for 30 min. On alternate days the mice received the other treatment and were placed into the opposing chamber. Each mouse thus received 4 drug and 4 saline pairings. Drug pairing was counterbalanced through the groups to both preferred and non-preferred sides as was the first day of administration of the drug. Saline/saline controls were included in the experimental design.

### Post-conditioning:

The guillotine doors were raised and the mice again placed into the central chambers and allowed free access to all three sections for 15 min. The position of each mouse was monitored and the time spent in each outer chamber was measured in s.

Seven groups were incorporated into the design:

| | |
|---|---|
| Group 1 | saline/saline controls (n = 10) |
| Group 2 | *d*-amphetamine sulphate (1.25 mg/kg, s.c.; n = 7) |
| Group 3 | *d*,*l*-MPH (10 mg/kg, s.c.; n = 10) |
| Group 4 | *d*-MPH (5 mg/kg, s.c.; n = 8) |
| Groups 5-7 | /-MPH (6.25 mg/kg s.c., 12.5 mg/kg s.c. or 25 mg/kg s.c.; n = 12, 9 and 10) |

### Antagonism Study

Groups of mice (n = 9-10/group) received saline or /-MPH (25 mg/kg, s.c.) followed 20 min later by saline, *d*-MPH (2.5 mg/kg, s.c.) or *d,l*-MPH (5 mg/kg, s.c.). Locomotor activity was measured in individual photocell boxes during the subsequent 60 min period as described in detail previously.

Five groups (n = 9-10/treatment group) were used:

| | |
|---|---|
| Group 1 | saline + saline controls: (n = 10) |
| Group 2 | saline + *d*-MPH (2.5 mg/kg, s.c.); n = 10 |
| Group 3 | saline + *d,l*-MPH (5 mg/kg, s.c.); n = 10 |
| Group 4 | /-MPH (25 mg/kg, s.c.) + *d*-MPH (2.5 mg/kg, s.c.); n = 10 |
| Group 5 | /-MPH (25 mg/kg s.c.) + *d,l*-MPH (5 mg/kg s.c.); n = 9 |

### Statistical Analysis

### Preliminary Study:

The total number of counts/time period was recorded and the data analysed by one-way analysis of variance with post-hoc t-test.

### CPP Study:

The time spent (s) by individual mice in the outer chambers pre- and post-conditioning were compared. Data were analysed by two-way analysis within subject analysis of variance followed by *post-hoc* t-test analysis.

### Antagonism Study:

The total number of counts/time period was recorded and the data analysed by one-way analysis of variance with Dunnett's t-test for multiple comparison against a single control.

From any analysis, p<0.05 was taken to be significant.

### CPP Study Results

### Pre-Conditioning:

Individual mice were exposed to the apparatus on each of three pre-conditioning test days. The initial preference for each mouse was calculated from the time spent (s) in each of the two outer chambers of the apparatus (mean ± sem) From this the mice were allocated to 1 of the 7 treatment groups in a design which was balanced to take account of preferred/non-preferred chambers and order of drug administration.

### Conditioning:

On each day mice received saline or drug and were exposed to one chamber of the apparatus. A daily record of individual body weights was also maintained.

### Post-conditioning:

The time spent (s) by individual mice in the three chambers of the apparatus was recorded. A comparison of the time spent before (pre) and after (post) the conditioning phase revealed that whilst saline/saline treatment did not cause any change in preference for a particular chamber (250.7 ± 21.5 s to 196.3 ± 22.0 s), *d*-amphetamine (1.25 mg/kg, s.c.) significantly increased the time spent in the drug-paired chamber (250.8 ± 7.9 s to 401.4 ± 35.3 s), consistent with a preference response.

Treatment with d-MPH (5.0 mg/kg, s.c.) increased preference for the drug-paired chamber (247.6 ± 16.9 s to 357.6 ± 51.0 s) whilst d,/.-MPH (10 mg/kg, s.c.) failed to produce a preference response (257.0 ± 16.3 s to 297.1 ± 26.3 s). Treatment with/-MPH(6.25, 12.5 or 25 mg/kg, s.c.) only produced a significant preference for the drug-paired chamber at the lowest dose tested whilst other doses failed to affect the response (270.5 ± 20.3 to 472.5 ± 35.2 s; 281.2 ± 25.5 to 322.8 ± 45.9 s; 275.7 ±16.1 to 323.7 t 47.7 s, respectively).

### Antagonism Study Results

Treatment with d-MPH (2.5 mg/kg, s.c.) or *d,l-*MPH (5 mg/kg, s.c.) significantly elevated LMA over a 60 min period when compared to saline-treated controls (1098.7 ± 106.7 increased to 2240.7 ± 259.2 and 3006.7 ± 149.7 counts/60 min respectively). Pretreatment with *l*-MPH (25 mg/kg, s.c.) failed to modify the effect of *d*-MPH (LMA increased to 2655.5 ± 165.6 counts/60 min); however the effect of *d,l*-MPH whilst remaining elevated compared to saline/saline-treated controls was significantly reduced (to 1961.2 ± 156.6 counts/60 min).

### Study 3 (Clinical)

A pilot study in 8 children with ADHD previously shown to be non-responsive to *d*,/-MPH was conducted. Any medications which might have interfered with the assessment of efficacy, safety and tolerability of the study drug were withdrawn for an appropriate period before the study. Two dose strengths of d-MPH were given as a single dose on separate study days one week apart, i.e. study days 1 (dose = 7.5 mg) and 8 (dose = 15 mg). Efficacy measurements were assessed post-dose relative to pre-dose on each of study days 1 and 8 and consisted of:
- a clinically relevant response in the investigator's opinion
- improvements in the 10-item Conners' global index scale (GIS)
- improvements in the clinical global impression scale (CGI)

The GIS is a validated scheme for diagnosing ADHD which is recognised worldwide. Relevant references are:
Conners CK, Conners' rating scales manual: Conners' teacher rating scale: Conners' parent rating scale: instruments for use with children and adolescents, Toronto, North Tonawanda, NY: Multi-Health Systems Inc, 1990; and
Conners CK, CRS-R. Conners' rating scale revised: instruments for use with children and adolescents, Toronto North Tonawanda, NY: Multi-Health Systems Inc, 1997.

This scheme is based on observation and rating of 10 behaviours which are:
- Restless or overactive
- Disturbs other children
- Excitable, impulsive
- Demands must be met immediately, easily frustrated
- Fails to finish things he/she starts
- Inattentive easily distracted
- Cries often and easily
- Temper outbursts
- Mood changes quickly and drastically
- Fidgeting

A reduction in the score is indicative of a clinical improvement.

The CGI is an established clinical measure of the severity of the illness, in the form of a score capable of detecting any change following treatment, and hence, providing an assessment of efficacy.

The results (Tables 2 to 5) show that there was an improvement in all three measures and that, in the case of the GIS and CGI, it was dose-related.

**Table 2**

| Investigator's Opinion | | |
|---|---|---|
| Subject | Study Day 1 | Study Day 8 |
| 1 | Y | Y |
| 2 | N | N |
| 3 | Y | Y |
| 4 | N | N |
| 5 | Y | Y |
| 6 | Y | Y |
| 7 | Y | Y |
| 8 | N | Y |
| Total Y | 5 | 6 |
| Total N | 3 | 2 |
| Y = Yes - clinically relevant response | | |
| N = No - no response | | |

**Table 3**

| Conners' Global Index Scale | | | | |
|---|---|---|---|---|
| Subject | Day 1 | | Day 8 | |
| | Pre-dose | Post-dose | Pre-dose | Post-dose |
| 1 | 13.5 | 5.5 | 12.5 | 7 |
| 2 | 5.5 | 6 | 7.5 | - |
| 3 | 17 | 12.5 | 16 | 6 |
| 4 | 12.5 | 5.5 | 9.5 | 5 |
| 5 | 13.5 | 8 | 11.5 | 6.5 |
| 6 | 11 | 7.5 | 12 | 7.5 |
| 7 | 14.5 | 10 | 12.5 | 7 |
| 8 | 8 | 6.5 | 9.5 | 6 |
| Total | 95.5 | 61.5 | 91 | 45 |
| Mean | 11.9 | 7.7 | 11.4 | 6.4 |
| Median | 13.0 | 7.0 | 11.8 | 6.5 |

**Table 4**

| Clinical Global Impression Scale Severity of Illness | | | | |
|---|---|---|---|---|
| Subject | Day 1 | | Day 8 | |
| | Pre-dose | Post-dose | Pre-dose Post-dose | |
| 1 | 5 | 3 | 5 | 2 |
| 2 | 2 | 1 | 1 | 1 |
| 3 | 6 | 5 | 6 | 2 |
| 4 | 3 | 1 | 1 | 1 |
| 5 | 5 | 3 | 5 | 2 |
| 6 | 5 | 3 | 5 | 2 |
| 7 | 5 | 3 | 5 | 2 |
| 8 | 3 | 3 | 3 | 3 |
| Total | 34 | 22 | 31 | 14 |
| Mean | 4.3 | 2.8 | 3.9 | 1.8 |
| Median | 5.0 | 3.0 | 5.0 | 2.0 |

**Table 5**

| Changes from baseline in CGI | | | | | | |
|---|---|---|---|---|---|---|
| Dose (mg) | Mean | SD | N | Median | Minimum | Maximum |
| 7.5 | -4.25 | 2.78 | 8 | -4.5 | -8 | 0.5 |
| 15 | -5.50 | 2.10 | 7 | -5 | -10 | -3.5 |

The change in the CGI was calculated for the pre-dose and post-dose measurements and the change from baseline then calculated. Analysis by a paired t-test shows that there was a statistically significant effect at both doses, the p values being 0.0034 and 0.0004 for the 7.5 and 15 mg doses, respectively

### Conclusions

The CPP data confirm that *d*-amphetamine and *d*-MPH produce a preference response in the murine conditioned place preference paradigm although, in contrast to a previous study, *d,l*-MPH was ineffective at the dose used. The lack of effect with *d,l*-MPH may reflect an inversion of the dose-response curve of this compound, a profile of effect suggested but not confirmed in the earlier study.

The data also suggest that *l*-MPH is capable of producing a preference response but only at the lowest dose used, higher doses being ineffective. The apparent inverted dose-response relationship observed with *l*-MPH may reflect a behavioural disruption effect of /-MPH which has been suggested elsewhere (Wilson *et al,* Psychopharmacologia (Berl.) 22:271-281 (1971) with higher doses of the racemate. However, the data from the present study do not fully support this hypothesis as no significant place aversion was obtained.

The preliminary study which examined a potential antagonist action of *l*-MPH against the effects of *d*-MPH and *d,l*-MPH on locomotor activity revealed that *l*-MPH was able to reduce but not reverse the stimulant actions of the racemate but did not affect the *d*-MPH induced stimulation of LMA. Given that the study was performed at one, high dose of *l*-MPH and at one time interval only, the conclusions to be drawn are speculative but would suggest that high doses of *l*-MPH may act as an antagonist at the dopamine transporter.

One other hypothesis to be considered relates to the potential partial agonist properties of *l*-MPH. Firstly, from the dose ranging study, evidence suggests that *l*-MPH may elevate LMA. Secondly, a low dose but not high doses of *l*-MPH induce a CPP. Finally, a high dose of *l*-MPH appears to block the effects of *d,l*-MPH. This profile of action may reflect partial agonist properties.

Children with ADHD previously shown to be non-responsive to racemic methylphenidate showed an improvement in the three clinical measures described above in response to *d-threo*-MPH. Therefore, in a clinical situation, it is reasonable to suppose that children who did not respond to current therapy in the form of Ritalin® may be successfully treated with *d-threo*-MPH.

## Claims

1. Use of *d-threo*-methylphenidate, in an enantiomeric excess of at least 90% with respect to its antipode, for the manufacture of a medicament for the treatment of a condition selected from attention-deficit hyperactivity disorder (ADHD), cancer treated with narcotic analgesics, depression, compulsive shopping disorder, narcolepsy and hypersomnia, in a patient who does not respond to treatment with racemic methylphenidate.

2. Use according to claim 1, wherein the condition is ADHD.

## Patentansprüche

1. Verwendung von *d-threo*-Methylphenidat in einem enantiomeren Überschuss von mindestens 90% bezüglich seines Antipoden für die Herstellung eines Medikaments zur Behandlung eines Zustandes, der aus dem hyperkinetischen Syndrom des Kindes (ADHD), Krebs, der mit narkotischen Analgetika behandelt wird, Depression, Zwangskaufstörung, Narkolepsie und Hypersomnie ausgewählt ist, bei einem Patienten, der nicht auf eine Behandlung mit racemischem Methylphenidat anspricht.

2. Verwendung nach Anspruch 1, bei der der Zustand ADHD ist.

## Revendications

1. Utilisation du d-thréo-méthylphénidate, en un excès énantiomérique d'au moins 90% par rapport à son antipode, pour la préparation d'un médicament pour le traitement d'un état choisi parmi un désordre d'hyperactivité avec déficit d'attention (ADHD), le cancer traité par des analgésiques narcotiques, la dépression, le trouble d'achat compulsif, la narcolepsie et l'hypersomnie, chez un patient qui ne répond pas au traitement avec le méthylphénidate racémique.

2. Utilisation selon la revendication 1, où l'état est le ADHD.
